# EUROPEAN PATENT SPECIFICATION

(11) **EP 2 979 671 B1**
(45) Date of publication and mention of the grant of the patent: **27.05.2020**
(21) Application number: 14179567.4
(22) Date of filing: 01.08.2014
(51) Int. Cl.: A61F 13/475, A61F 13/533, A61F 13/536, A61F 13/53

(54) **Array of absorbent articles having channel-forming areas**
Anordnung absorbierender Artikel mit Kanalbildungsbereichen
Réseau d'articles absorbants comportant des zones de formation de canal

(43) Date of publication of application: 03.02.2016
(73) Proprietor: The Procter & Gamble Company, Cincinnati, OH 45202 (US)
(72) Inventor: Kreuzer, Carsten Heinrich, 65824 Schwalbach am Taunus (DE); Bianchi, Ernesto Gabriel, 65824 Schwalbach am Taunus (DE); Rinnert, Thorsten, 65824 Schwalbach (DE)
(74) Representative: P&G Patent Germany

(56) References cited:
- EP-A1- 2 740 449
- US-A1- 2005 137 543
- US-A1- 2006 069 371
- US-A1- 2007 244 455
- US-A1- 2010 022 978
- US-B2- 6 648 864

## Description

### FIELD OF THE INVENTION

The invention relates to personal hygiene absorbent articles of the type used by an incontinent individual to absorb body exudates in particular urine. The articles include in particular baby and toddler diapers (including training pants), feminine sanitary pads and adult incontinence articles.

### BACKGROUND OF THE INVENTION

These personal hygiene absorbent articles typically comprise an absorbent material disposed between a fluid-permeable skin-facing sheet (topsheet) and a fluid-impermeable garment-facing sheet (backsheet). Superabsorbent polymer ("SAP") materials capable to absorb several time their own weight of urine are nowadays typically used as absorbent material. SAP particles are typically mixed with cellulose fluff, but cellulose-free absorbent cores (so-called "airfelt-free" absorbent core) have been more recently commercialized. The absorbent material may be typically enclosed in a discrete component called absorbent core but it can also be more simply deposited directly between the topsheet and backsheet.

Personal hygiene absorbent articles are typically offered in different sizes to accommodate the particular needs of different wearers. For example, baby care diapers are typically segmented according to the weight of the wearer, each diaper size corresponding to a range of weight of the baby. The size of each articles increases with the weight ranges. The number of sizes offered and the corresponding weight ranges may vary between manufacturers and markets. Similar segmentation also exists in other categories of personal hygiene articles, such as feminine care and adult incontinence articles, and may be related to the severity of the incontinence as well as the size of the wearer.

Typically, the dimensions of the absorbent core and the amount of absorbent material increase with the size of the articles, in particular to accommodate larger quantity of urine, at least as long as the wearer remains incontinent. However articles for older children in the training phase (e.g. training pants) which are starting to be clean may comprise less absorbent material than product for younger children. The overall design and proportions of the absorbent core however generally do not considerably vary with the product sizes.

Recently, it has been proposed to provide absorbent structures that comprise superabsorbent polymers, optionally a cellulosic material, and at least a pair of substantially longitudinally extending absorbent material free zones that can form channels. Examples of such structures are disclosed in WO2012/170778 (Rosati et al., see also WO2012/170779, WO2012/170781 and WO2012/170808) and further in WO2014/093319, WO2014/093311 (both Arizti), WO2014/093310 (Ehrnsperger). The material free zone in these disclosures may be curved. Other prior art discloses channels which are straight, and typically oriented in the longitudinal and/or transversal direction of the core, as in WO95/11652 (Tanzer) or WO2012/052,172A1 (Van Malderen).

WO2008/023291 (Cohen) discloses that children at different stages of development have various predominant leg positions. Cohen addresses the different predominant leg positions of newborns, infants, and toddlers by providing an array of disposable articles comprising different leg opening positions. However Cohen does not disclose adapting the construction of the absorbent cores according to the stage of development of the wearer. US2005/0137543A1 discloses a series of toilet training pants corresponding to different stages of toilet training.

US 2006/069371 discusses a disposable absorbent pad with paired grooves which, as measured in the direction along the longitudinal axis, may be appropriately selected depending on a size of the individual absorbent pad to provide an improved fit to a pad wearer's body.

There is a permanent need to improve fit and comfort of absorbent articles. Providing absorbent articles of different dimensions (in longitudinal and transversal direction) based on the baby weight to provide the right coverage and performance is known. The present inventors have now found that the dimensions of the channel-forming areas should be specifically adapted to the size of the wearer. Adapting the dimensions and in particular the ratio of curvature of the channel-forming areas to the baby's morphological changes (height, weight and leg circumference) is beneficial to enable proper fit along the full diapering age time. In addition, the inventors have found a correlation between channels dimensions and the babies' morphology parameters (i.e. like leg circumference) that can be used to optimize fit for a given size. In short, the geometry of the channel-forming areas should be adapted to the size of the wearer to provide a better fit.

### SUMMARY OF THE INVENTION

The invention according to claim 1, directed to an array of absorbent articles comprising at least two absorbent articles of different sizes. Each article has a wearer-facing side, a garment-facing side and an absorbent core between the wearer-facing side and the garment-facing side. Each absorbent article further has a longitudinal axis, a front region, a back region and a crotch region. The absorbent core comprises a pair of channel-forming areas at least partially present in the crotch region of the article, wherein these channel-forming areas are symmetrically disposed on opposite side of the longitudinal axis and are concave towards the longitudinal axis, wherein each channel-forming area has a length (CL) and a channel height (CH). The ratio of the length to ratio (CL/CH) of the channel-forming areas increases with the sizes of the article to provide an improved fit. The absorbent material of each article is enclosed by a core wrap and the channel-forming areas are formed by attaching the top side of the core wrap to the bottom side of the core wrap along a core wrap bond, and wherein the channel-forming areas comprise an area substantially free of absorbent material which is surrounded by absorbent material and through which the core wrap bond is formed.

### BRIEF DESCRIPTION OF THE DRAWINGS

Fig. 1 shows a top view of an exemplary absorbent article in the form of a taped diaper, with inner layers partially revealed, and comprising an absorbent core with a pair of curved channel-forming areas;
Fig. 2 shows a top view of the absorbent core of Fig. 1 in isolation;
Fig. 3a shows a transversal cross-section of the core of Fig. 2;
Fig. 3b shows the same transversal cross-section of the core of Fig. 3a after core has absorbed a fluid and swollen;
Fig. 4 shows a close-up section of the central part of one channel-forming area;
Fig. 5a,b,c schematically illustrate different absorbent cores with different channel-forming areas that can be used to make an array of articles according to the invention;
Fig. 6a,b,c is as Fig. 5a,b,c with alternative channel-forming areas;
Fig. 7 shows a core with alternatively shaped channel-forming areas;
Fig. 8 shows a core with alternatively shaped channel-forming areas;
Fig. 9 is a chart showing the relation between Rise and Leg Circumferences (in mm) as a function of weight (in kg).

### DETAILED DESCRIPTION OF THE INVENTION

### General description of an absorbent article

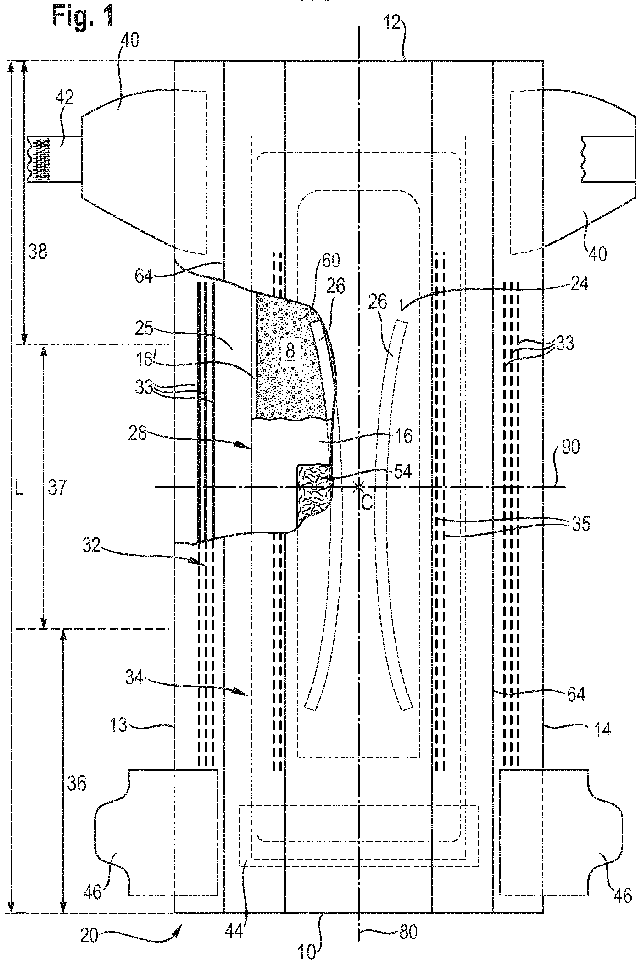

An exemplary absorbent article according to the invention in the form of a baby taped diaper 20 is represented in Fig.1. Fig. 1 is a top plan view of the exemplary diaper 20, in a flat-out state, with portions of the structure being cut-away to more clearly show the construction of the diaper. This diaper 20 is shown for illustration purpose only. The absorbent article can also be for example a pant-type article with pre-formed side seams. Unless otherwise indicated, dimensions and areas disclosed herein apply to the article in this flat-out configuration. If some part of the article is under tension due to elasticized components, the article may be typically flattened using clamps along the periphery of the article and/or a sticky surface, so that the topsheet and backsheet can be pulled taut so as to be substantially flat. Closed articles such as training pant may be cut open along the side seams to apply them on a flat surface.

The absorbent article 20 comprises a front edge 10, a back edge 12, and two longitudinally extending side (lateral) edges 13, 14 joining the front edge and the back edge. The front edge 10 is the edge of the article which is intended to be placed towards the front of the user when worn, and the back edge 12 is the opposite edge. The absorbent article is notionally divided by a longitudinal axis 80 extending from the front edge to the back edge of the article and dividing the article in two substantially symmetrical halves relative to this axis, when viewing the article from the wearer facing side in a flat out configuration, as exemplarily shown in Fig. 1. This axis 80 may typically be concomitant with the longitudinal axis 80' of the absorbent core. The article has a length L as measured along the axis 80 from the back edge to the front edge. The absorbent article 20 can also be notionally divided by a transversal axis 90 into a front region and a back region of equal length measured on the longitudinal axis, when the article is in such a flat state. This article's transversal axis 90 is perpendicular to the longitudinal axis 80 and placed at half the length of the article. The intersection of the longitudinal axis and transversal axis are referred herein as the Crotch Point "C".

The absorbent article is further notionally divided in a front region, 36, a back region 38 and a crotch-region 37 in between. The front region 36 is defined as the region of the article extending from the front edge 10 and having a length of a third of L along the longitudinal axis 80. The back region 38 is defined as the region of article extending from the back edge 12 of the article and having a length of one third of L along the longitudinal axis 80. The crotch region 37 is the intermediate region between the front and back regions, and also having a length of a third of L along the longitudinal axis 80.

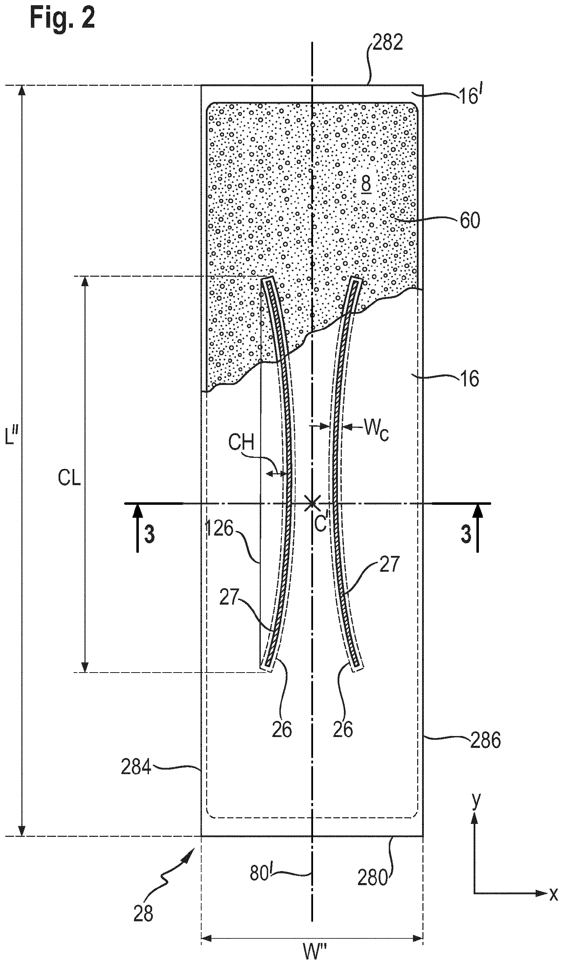

The absorbent article 20 comprises a wearer-facing side, which may be principally formed by a liquid permeable topsheet 24, a garment-facing surface which may be formed by a liquid impermeable backsheet 25, and an absorbent core 28 between the topsheet 24 and the backsheet 25. The absorbent core 28 is shown in isolation in Fig. 2. The topsheet 24, the backsheet 25, the absorbent core 28 and the other article components may be assembled in a variety of well-known configurations, in particular by gluing and/or heat embossing. Exemplary diaper assemblies are for example generally described in US 3,860,003, US 5,221,274, US 5,554,145, US 5,569,234, US 5,580,411, and US 6,004,306. The absorbent article is preferably thin, for example with a caliper of from 2.0 mm to 8.0 mm, in particular from 3.0 mm to 6.0 mm, a the crotch point as measured using the Absorbent Article Caliper Test described below.

The article 20 may also comprise further components such as an acquisition layer and/or a distribution layer (collectively referred to as acquisition-distribution system "ADS", designated as 54), and elasticized gasketing cuffs 32 present between topsheet and backsheet and upstanding barrier leg cuffs 34. The ADS may be profiled and/or curved towards the back edge of the article, for example as disclosed in WO2014/093323. Fig.1 also shows other typical taped diaper components such as a fastening system comprising fastening tabs 42 attached towards the back edge 12 of the article and cooperating with a landing zone 44 towards the front edge 10 of the article. These fastening features are typically absent from pant-type articles which have a pre-formed side seam. The absorbent article may also comprise other typical components, which are not represented in the Figures, such as a back elastic waist feature, a front elastic waist feature, transverse barrier element, a wetness indicator that changes appearance when contacted with urine, a lotion application, etc. These components are well-known in the art and will not be further discussed herein. Reference is made to WO2014/093310 where several examples of these components are disclosed in more details.

Typically, adjacent layers will be joined together using conventional bonding method such as adhesive coating via slot coating, spiral gluing, or spraying on the whole or part of the surface of the layer, or thermo-bonding, or pressure bonding or combinations thereof. Most of the bonding between components is for clarity and readability not represented in the Figure. Bonding between the layers of the article should be considered to be present unless specifically excluded. Adhesives may be typically used to improve the adhesion of the different layers, for example between the backsheet and the core wrap a disclosed in WO2012/170341A1. The adhesives used may be any standard hotmelt glue as known in the art. The absorbent core 28 and the channel-forming areas 26 will now be further described.

### General description of the absorbent core 28

As used herein, the term "absorbent core" refers to an individual component of the absorbent article and which comprises an absorbent material enclosed in a core wrap. The absorbent core as defined herein does not include the acquisition-distribution layer or multilayer system if present. The core wrap is typically formed by one or two layers of nonwoven or tissue materials, but it is not excluded that for some articles of simple construction the core wrap may be partially or entirely formed by the topsheet and/or the backsheet. The absorbent core is typically the component of an absorbent article that has the most absorbent capacity of all the components of the absorbent article and which comprises all, or at least the majority of, superabsorbent polymer (SAP). The core may consist essentially of, or consist of, the core wrap, the absorbent material and optionally adhesives. The terms "absorbent core" and "core" are herein used interchangeably.

The absorbent cores of the invention are substantially planar. By substantially planar, it is meant that the absorbent core can be laid flat on a planar surface. The absorbent cores may also be typically thin and conformable, so that they can also be laid on a curved surface for example a drum during the making process, or stored and handled as a continuous roll of stock material before being converted into an absorbent article.

For ease of discussion, the exemplarily absorbent core of Figs. 2-3 is represented in a flat state. The absorbent core is relatively thin relative to its other dimensions in the transversal direction (x) and the longitudinal direction (y). These directions correspond to the transversal and longitudinal direction of the article respectively. Unless otherwise indicated, dimensions and areas disclosed herein apply to the core in this flat-out configuration. The same applies to an absorbent article, as exemplarily represented in Fig. 1 as a taped diaper, in which the core is integrated. The absorbent cores and articles of the invention are discussed with reference to the Figures and the numerals referred to in these Figures; however these are not intended to limit the scope of the claims unless specifically indicated.

The absorbent cores 28 illustrated comprise a front edge 280, a back edge 282 and two longitudinal side edges 284, 286 joining the front edge and the back edge. The front edge of the core is the edge intended to be placed towards the front edge of the absorbent article in which the core is or will be integrated. Typically the front and back edges 280, 282 of the core may be shorter than the longitudinal side edges 284, 286 of the core. The absorbent core also comprises a top side 288 and a bottom side 290. The top side of the core is placed or intended to be placed towards the wearer-facing side (topsheet 24) of the article and the bottom side is the side placed or intended to be placed towards the garment-facing side (backsheet 25) in the finished article. The top side of the core wrap may be more hydrophilic than the bottom side, for example after treatment with a wetting agent.

The absorbent core may be notionally divided by a longitudinal axis 80' parallel to the longitudinal direction y and extending from the front edge 280 to the back edge 282 and dividing the core in two substantially symmetrical halves relative to this axis, when viewing the core in the plane formed by the longitudinal and transversal direction (x, y). The length L" of the core is measured from the front edge 280 in direction of the back edge 282 along the longitudinal axis 80', including the region of the core wrap which does not enclose the absorbent material, in particular at the front and back end seals when present. The crotch point of the core C' is the point that is aligned vertically with the crotch point C of the absorbent article and normally also placed on the longitudinal axis 80' of the absorbent core.

The width W" of the core is the maximum dimension of the core wrap measured along the transversal direction x, which is perpendicular to y. The outline of the absorbent core defined by the core wrap can typically be generally rectangular. The width W" and length L" of the core may vary depending on the intended usage. The core wrap may also be shaped, with a width at the crotch point C' narrower than at the front and /or back of the core wrap. For baby care applications such as diapers and infant training pants for example, the width of the core may typically ranges from 4 cm to 22 cm and the length from 10 cm to 62 cm depending on the size and capacity desired. The ratio length to width (L"/W") may for example range from 2 to 10. Adult incontinence articles may typically be longer and larger than baby articles and have even higher dimensions.

The absorbent core comprises an **absorbent material 60** encompassed within the core wrap. The absorbent material may comprise a high proportion of superabsorbent polymer (herein abbreviated as "SAP"). The term "superabsorbent polymer" refers herein to absorbent materials, which may be cross-linked polymeric materials, and that can absorb at least 10 times their weight of an aqueous 0.9% saline solution as measured using the Centrifuge Retention Capacity (CRC) test (EDANA method WSP 241.2-05E). The SAP may in particular have a CRC value of more than 20 g/g, or more than 24 g/g, or of from 20 to 50 g/g, or from 25 to 40 g/g. The SAP content may represent at least 80% and up to 100% by weight of the absorbent material contained in the core wrap. The SAP may in particular be in particulate forms (SAP particles) but other forms are also possible, such as absorbent foam or fibers. Further detailed examples of absorbent material, in particular SAP are disclosed in WO2014/093310 (Ehrnsperger). In particular, the absorbent material may comprise or consist of SAP particles that require a time to reach an uptake of 20 g/g (T20) of less than 240 s as measured according to the K(t) test method described in WO2012174026.

The absorbent material may in particular be substantially free of cellulose fibers, meaning it comprises at least less than 20% by weight of cellulose fibers relative to the total weight of absorbent material, in particular less than 10%, or less than 5% and down to 0% by weight of cellulose fibers. The absorbent core may thus be relatively thin, in particular thinner than conventional cores comprising cellulosic fibers. In particular, the caliper of the core (before use) as measured at the crotch point (C') or at any other points of the surface of the core according to the Core Caliper Test as described herein may be from 0.25 mm to 5.0 mm, in particular from 0.5 mm to 4.0 mm.

The absorbent material 60 forms an absorbent material deposition area 8 within the core wrap. The deposition area 8 may comprise a continuous layer of absorbent material in the plane of the core, as shown in Figure 2. Alternatively, the deposition area may comprise discrete or joined land areas which comprise absorbent material and material free junction-areas in-between. The basis weight (amount deposited per unit of surface) of the absorbent material may also be varied to create a macroscopic profiled distribution of absorbent material in the longitudinal direction (y) and/or the transversal direction (x). Typically the absorbent material of the core may be advantageously distributed in somewhat lower amount towards the back portion 38 of the core as more absorbency is typically required towards the crotch region and front region. Other absorbent material distributions are however possible, for example a homogenous distribution across the absorbent material area 8 may be easier to make.

The absorbent material deposition area 8 may be generally rectangular, as illustrated in Fig. 2, but shaped absorbent material deposition area are also desirable. A typical shaped deposition area tapers in the crotch region, which has at least a point where its width is smaller than the width of the absorbent deposition material area at the front and/or back regions, in particular forming dog-bone or sand-hour shaped area absorbent material area. Further detailed examples of absorbent material distribution that can be used herein are disclosed in WO2014/093310 (Ehrnsperger).

Various absorbent core designs comprising high amount of SAP have been proposed in the past. The processes used can allow relatively precise deposition of SAP at relatively high speed, and which may be used to make the absorbent cores of the invention, see for example in US5,599,335 (Goldman), EP1,447,066 (Busam), WO95/11652 (Tanzer), US2008/0312617 (Hundorf), WO2012/052172 (Van Malderen) and in particular WO2012/170778 (Rosati et al., see also WO2012/170779, WO2012/170781 and WO2012/170808).

The absorbent material may be deposited for example using a SAP printing technology as disclosed in US2006/024433 (Blessing), US2008/0312617 and US2010/0051166A1 (both to Hundorf et al.). This technique uses a transfer device such as a printing roll to deposit SAP particles onto a substrate disposed on a grid of a support (e.g. a lay-on drum). The grid may include a plurality of cross bars extending substantially parallel to and spaced from one another so as to form ribs extending between the cross-bars. The SAP is deposited inside these ribs. This technology allows high-speed and precise deposition of SAP on a substrate in particular to provide areas substantially free of absorbent material surrounded by absorbent material through which the core wrap can be bonded to itself to form channel-forming areas. The areas substantially free of absorbent material can be formed for example by providing an area on the support where no SAP is applied and then bonding both sides of the core wrap to each other through these areas free of absorbent material. One way to achieve this is exemplary disclosed in US2012/0312491 (Jackels).

**The core wrap** may be formed by any substrate materials suitable for receiving and containing the absorbent material. Typical substrate materials used are in particular nonwovens, paper, tissues, films, wovens, or laminate of any of these. The core wrap may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US7,744,576, US2011/0268932A1, US2011/0319848A1 and US2011/0250413A1. Nonwoven materials provided from synthetic fibers may be used, such as PE, PET and in particular PP.

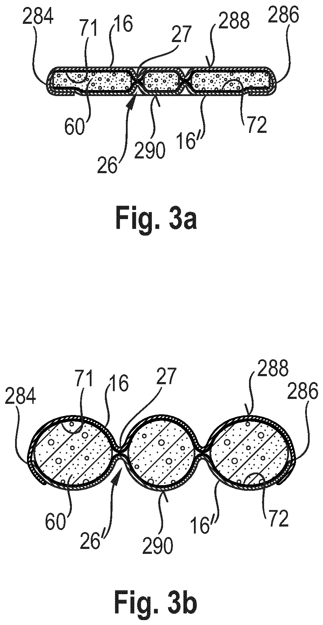

The core wrap may, as shown in the cross-sectional view of Fig.3, comprise a first substrate 16 forming the top side 288 of the core and a second substrate 16' essentially forming the bottom side 290 of the core. Alternatively, it is also known in the art to make a core wrap out of a single substrate, or to use the backsheet or topsheet as substrate to directly, partially or completely form the core wrap. When two substrates are used, the core wrap may have a C-wrap along each longitudinal side edges 284, 286 of the core. The core wrap is not considered as absorbent material for the purpose of calculating the percentage of SAP in the absorbent core. Examples of core wrap construction are further detailed in WO2014/093310.

The absorbent core may comprise one or more layers of glue to help immobilizing the absorbent material. The absorbent core 28 may comprise at least one **auxiliary glue** 71, 72 layer applied on the inner surface of the top side and/or the bottom side of the core wrap. The auxiliary glue may be applied directly over the substrate on which the absorbent material is subsequently deposited, thus at least partially immobilizing the absorbent material on the substrate. The auxiliary glue may also at least partially form the core wrap bond 27 of the channel-forming areas 26. The auxiliary glue may also be useful to improve the adhesion of a fibrous thermoplastic material, when present, to the substrate. The auxiliary glue can be applied by any adhesive applicator known in the field, in particular bead, slot or spray nozzles. For example, the auxiliary glue can be applied using a slot coating process as a pattern comprising a plurality of spaced-apart slots which may each extend in the longitudinal direction. The slots may for example have a width of from 0.5 mm to 3 mm, and/or have a lateral spacing there-between of from 0.5 mm to 4 mm.

The absorbent core 28 may also comprise **a fibrous thermoplastic adhesive material** (not shown), also known as microfiber glue, to help immobilizing the absorbent material 60 within the core wrap. The fibrous thermoplastic adhesive material may be applied, typically by spraying, over an absorbent material that has been discontinuously deposited on a substrate during the core making process, thus forming land and junction areas as indicated above. The fibrous thermoplastic adhesive material contacts the absorbent material and the substrate layer in the absorbent material free junction areas. This imparts an essentially three-dimensional net-like structure to the fibrous layer of thermoplastic adhesive material, which in itself is essentially a two-dimensional structure of relatively small thickness, as compared to the dimension in length and width directions. Thereby, the fibrous thermoplastic adhesive material may provide cavities to cover the absorbent material, and thereby immobilizes this absorbent material. A dual layer core can thus be constructed wherein the land areas of one layer correspond to the material-free junction areas of the other layer and vice versa, resulting in continuous dual absorbent layer.

More details about these glues and how they can be applied in a SAP printing process is further generally disclosed in US2006/024433 (Blessing), US2008/0312617 and US2010/051166A1 (both to Hundorf et al.) and US2014/027066A1. The absorbent cores may advantageously provide a sufficient immobilization of the absorbent material in dry and wet state. The absorbent core advantageously achieves an SAP loss of no more than about 70%, 60%, 50%, 40%, 30%, 20%, or 10% according to the Wet Immobilization Test described in US2010/051166A1.

### Channel-forming areas 26

Each absorbent core further comprises at least a pair of channel-forming areas 26 which will now discussed in more details. The channel-forming areas 26 may each comprise an absorbent material free area within which a core wrap bond 27 is present. The channel-forming areas may typically be not visible in the article before use because of the opacity of the other layers of the article, in particular when the absorbent core is substantially free of cellulose fibers and thus only a few mm thin. The thickness of the core 28 when dry, as represented in Fig. 3, is exaggerated to clearly show the channel-forming areas 26 and core wrap bond 27.

The channel-forming areas at least initially do not substantially swell as the absorbent core absorbs urine or another fluid, while the absorbent material adjacent the channel-forming areas can swell considerably. The channel-forming areas thus forms three-dimensional channels when the absorbent material adjacent the channel-forming areas absorbs a fluid and swells. As the core absorbs more liquid, the depressions within the absorbent core formed by the core wrap bond 27 between the two substrates become deeper and apparent to the eye and the touch. With sufficiently strong core wrap bond, and/or a relatively extensible substrate material, the channels can remain permanent until complete saturation of the absorbent material. On the other hand, the core wrap bonds 27 may be designed to at least partially open or break when the core is close to saturation so as not to unduly restrict the swelling of the absorbent material when the core is substantially loaded.

The channel-forming areas may be formed in different manners. In particular, channel-forming areas may be areas of the core that are substantially free of absorbent material and through which the top side of the core wrap is bonded to the bottom side of the core wrap. The core wrap bond 27 between the top side and bottom side of the core wrap in these areas may be at least partially formed by auxiliary glue 71, 72 and/or microfiber glue (not shown) applied directly to the inner surface of at least one of the substrate. This is exemplarily disclosed in WO2012/170778 for example. However it is also known to form bonds using other bonding means such as ultra-sonic bonding, pressure bonding or heat bonding. The channel-forming areas can also possibly not comprise such core wrap bonds, but the resulting channels may then be less resistant to compression and the absorbent material may fill the channel areas relatively quickly when the article is worn. The channel-forming areas may also be formed by a core wrap bond with minimal or no surrounding absorbent material free areas.

The channel-forming areas 26 are pair-wise symmetrically disposed on opposite side of the longitudinal axis of the article 80 and are concave towards the longitudinal axis 80 of the article. By "concave" towards the longitudinal axis" it is meant that the channels have a point or area which is closest to the longitudinal axis (typically in the crotch region of the article) and that the channels generally diverge from this closest point or area as the channels extend towards the front and back of the article. The smallest distance or gap between the pair of areas may be for example at least 5 mm, or at least 10 mm, or at least 16 mm and may for example be up to 40 mm.

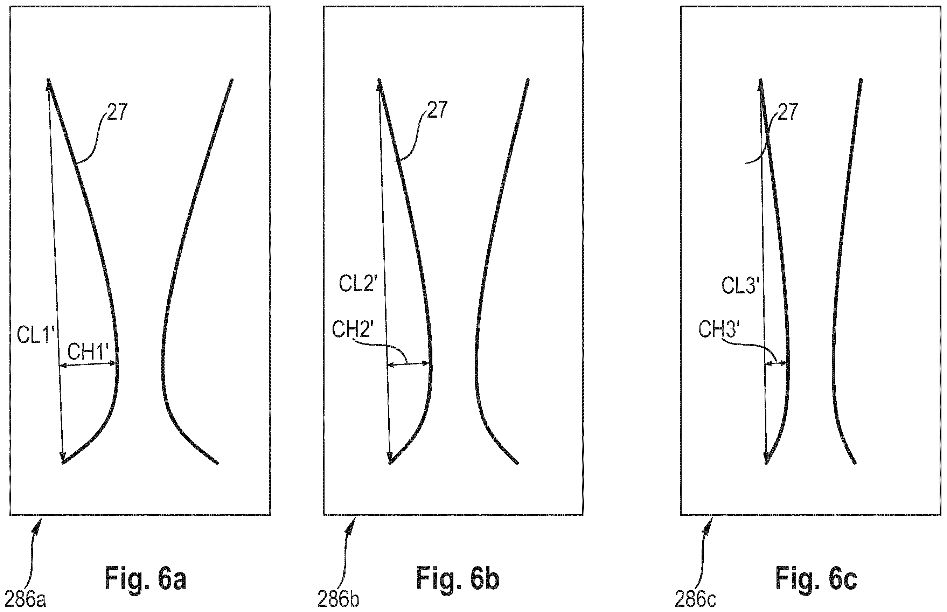

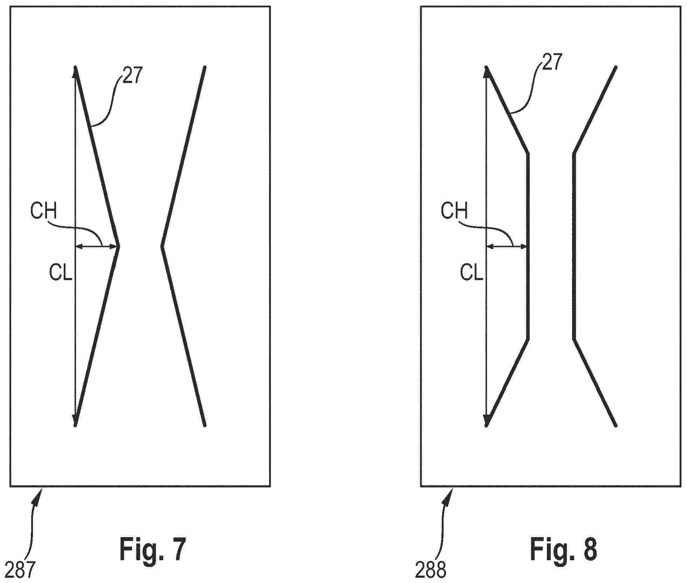

The channels may be advantageously curvilinear. The curve may have a substantially constant radius of curvature along the length of the curved portion as illustrated on Fig. 2 or this radius may vary as illustrated in Fig. 6a. It also not excluded that at least portion of the channels may be straight and/or comprise a plurality of straight segments. For examples, the channel-forming areas could be approximated by two lines meeting at the point closest to the longitudinal axis and forming together a flat V as illustrated on Fig. 7. The channel-forming areas may also comprise three or more segments, as illustrated on Fig. 8.

Although not represented in the Figures, it is not excluded that a portion of the channel-forming areas may not be concave towards the longitudinal axis, for example that a portion of the channels at the extremities toward the front and/or back edge of the core may be parallel to the longitudinal axis or even convex (converging towards the longitudinal axis). In this case, only the concave portion of the channel-forming areas is considered for calculating the length to height ratio (see below) of the channel-forming areas. The absorbent core may comprise further channel-forming areas in addition to the curved pairs, in particular channel-forming areas that may not be curved or that may placed in the front region of the article only as disclosed in WO2012/170779.

The channel-forming areas 26 may be substantially free of absorbent material, so that the bond 27 between the top side 288 and bottom side 290 of the core wrap can be easily formed within these areas substantially free of absorbent material, for example by gluing and/or pressure bonding these two surfaces together. By "substantially free of absorbent material" it is meant that there can be practically no absorbent material in these areas. Minimal amount such as involuntary contaminations with absorbent material particles that may occur during the making process are disregarded. Within the channel-forming areas, the top side 288 of the core wrap is attached to the bottom side 290 by a core wrap bond 27 as illustrated in Figs. 2-3. The portion of the channel-forming areas immediately adjacent the bond 27 may be substantially free of absorbent material. The channel-forming areas as a whole including this absorbent material free area may have a width Wc. In this example, the width of the channel-forming area 26 which corresponds to an area substantially free of absorbent material may be of about 8 mm and the channel bond 27 may be of about 2 to 3 mm. The channel-forming areas may be substantially free of absorbent material along at least part of their length across a width Wc which is at least 2 mm, or at least 3 mm or at least 4 mm, up to for example 20 mm, or 16 mm or 12 mm. The width Wc of the areas substantially free of absorbent material may be constant through substantially its whole length or may vary along the length of the channel-forming areas.

The channel-forming areas 26 and bonds 27 are advantageously substantially surrounded by the absorbent material 60, when considering the plane of the core. In particular they may advantageously not extend to any of the edges of the absorbent material deposition area to reduce the risk of leakage. Typically, the smallest distance between a channel-forming area and the closest edge of the core wrap may be at least 5 mm or advantageously at least 10 mm.

When the absorbent material 60 swells upon absorbing a fluid, the core wrap bonds 27 remain at least initially attached in the channel-forming areas 26. The absorbent material 60 having swollen in the rest of the core, the core wrap forms channels, i.e. elongated depressions, along the core wrap bond 27, as illustrated in Fig. 3b. These channels 26' are three-dimensional and have been found to function as bending lines which drive the bending of the diaper to conform to the wearer's anatomy and therefore improve the fit of the diaper. The channels can also serve to distribute an insulting fluid along their length to a wider area of the core. They may provide a quicker fluid acquisition speed and a better utilization of the absorbent capacity of the core. The channels can also provide a deformation of an overlying layer such as a fibrous layer 54 and provide corresponding ditches in the overlying layer. The absorbent core may comprise other areas substantially free of absorbent material, such as the spaces between the absorbent material areas, but without a core wrap bond, these non-bonded areas will typically not form durable three-dimensional channels.

The core wrap bond 27 may also be designed to gradually open in a controlled manner when exposed to a large amount of fluid. The bonds may thus remain substantially intact at least during a first phase as the absorbent material absorbs a moderate quantity of fluid. In a second phase the core wrap bonds 27 in the channels can start opening to provide more space for the absorbent material to swell while keeping most of the benefits of the channels such as increased flexibility of the core in transversal direction and fluid management. In a third phase, corresponding to a very high saturation of the absorbent core, a more substantial part of the channel bonds can open to provide even more space for the swelling absorbent material to expand. The strength of core wrap bond 27 within the channels can be controlled for example by varying the amount and nature of the glue used for the attaching the two sides of the core wrap, the pressure used to make the core wrap bond and/or the distribution of the absorbent material, as more absorbent material will usually causes more swelling and will put more pressure on the bond. The extensibility of the material of the core wrap may also play a role.

The core wrap bond 27 may be continuous along each channel-forming area 26 but it may also be discontinuous (intermittent) such as formed by series of point bonds. Each channel-forming areas 26 is also advantageously continuous but it is not excluded that it may be comprised of discrete section separated by small gaps as long as a generally concave outline is formed by the discrete sections. An auxiliary glue 71, 72 when present may at least partially help forming the bond 27. Typically, some pressure can be applied on the substrates in the areas 26 so that the auxiliary glue better forms the bonds between the substrates. Of course it is not excluded that the core wrap bond 27 is made via other known attachment means, such as pressure bonding, ultrasonic bonding or heat bonding or combination thereof, in which case the width of the channel-forming areas may be even more reduced. If an auxiliary glue 71, 72 is applied on the inner surface of any of the substrates 16, 16' as a series of longitudinally-oriented continuous slots, the width and frequency of these slots may advantageously be such that at least one slot of auxiliary glue is present at any level of the channel in the longitudinal direction. For example the slots may be 1 mm wide with a 1 mm distance between neighboring slots, and the absorbent material free areas forming the channel-forming areas have a width of about 8 mm. In this example, 4 slots of auxiliary glue will be present on average in each of the areas 26.

In general, the core wrap bonds 27 may have the same outline but be slightly smaller than the absorbent material free areas in which they are formed due to the tolerance required in some manufacturing process.

The channel-forming areas 26 are at least partially present in the crotch region 37 of each article; in particular they can be sufficiently elongated to extend through the whole of the crotch region and into the front region and/or the back region of the article. The channel-forming areas 26 may thus have a length CL which is at least 25% of the length L of the article, in particular at least 33% or even at least 40% of L, up to 90% or 80% of L.

The channel-forming areas 26 are further characterized herein by the ratio of their channel length (CL) to the channel height (CH). The channel length (CL) corresponds to the distance between the two extremities of the channel-forming areas, as illustrated in Fig. 2 by the line 126, including the absorbent-material free area if present. If the channel-forming areas have a measurable width Wc, the center of the channel-forming areas at the extremities is used to measure the channel length.

The straight line 126 joining these two extremities may be generally parallel to the longitudinal axis 80' of the core (see Fig. 2) but it is not excluded that this line may have an angle relative to the longitudinal axis if one of the extremities is skewed transversally relative to the other extremity. Such an angle may for example be up to 25°, or up to 20° relative to the longitudinal axis 80 of the article or the longitudinal axis 80'of the absorbent core.

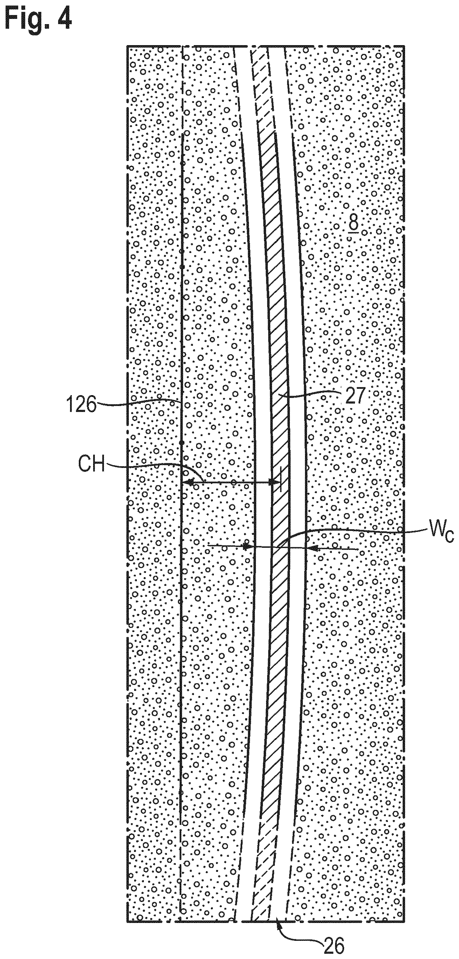

The channel height (CH) is the largest distance between the line 126 joining the two extremities of the channel-forming areas and the rest of the channel-forming area, as measured perpendicularly to the joining line 126. This is illustrated in Fig. 4, which is a close-up of the relevant part of Fig. 2 where the distance CH is measured. If the channel forming-areas 26 have a measureable width, as is illustrated, the channel height is measured by referring to the central portion of the channel-forming area 26, which may typically be the center of the bond 27, as shown on Fig. 4.

The inventors have found that among an array of articles of different sizes having concave channel-forming areas, the ratio CL/CH should increase with the sizes of the article to provide an optimized fit for at least two of the articles within the array, in particular at least three of the articles. This will be further discussed in the next section.

### Array of absorbent products

The present invention is directed to on an array of absorbent articles comprising at least two different articles, in particular at least three articles, comprising channel-forming areas as indicated previously and wherein the CL/CH ratio of the articles increases with the size of the articles. The article's size may be typically marked directly on the absorbent article, for example printed on the backsheet, and will also typically be marked on the packaging of the absorbent articles to facilitate selection by the user. The "user" as used herein may be the caregiver for baby care articles or the wearer itself for adult incontinence articles. The articles in the array are typically part of a line-up or system of absorbent articles that covers a range of different article sizes. The array of absorbent articles may typically be marketed under a common primary designation, usually a registered trademark such as Pampers@ or Luvs® (both trademarks of the Procter & Gamble Company). The articles of the array may also be marketed under a secondary designation, which may be the same or different for each articles in the array, for example Baby-Dry™ or Cruisers™.

The sizes of the articles may be directly linked to the weight of the intended users, typically as a weight range. For example, the article's sizes for baby care products may be indicated by a number (size 0, 1, 2, ..6), a letter (XS, S, M, L, XL..), a short description (Newborn, mini, midi, maxi, ...) or a combinations of these. Typically, the amount of absorbent material increases with the size of the articles, and advantageously the length and width of the articles as well. The article's size may thus typically increase with the weight of the wearer, but it is also possible that the article's size may be additionally or alternatively linked to the ages or the urine absorption needs of the wearer.

Since the needs of the users may vary across regions, the same size designation may be used for articles with different weight ranges by the manufacturer. For baby care articles for example, the following table gives the different size numbers to weight ranges (in kg) indicated for baby diapers sold at the time of filing under the Pampers@ Baby-Dry™ designation for different geographies.

| **Region** | **Size** | | | | | |
|---|---|---|---|---|---|---|
| | **1** | **2** | **3** | **4** | **5** | **6** |
| **North America** | 4 - 6 | 5 - 8 | 7 - 13 | 10 - 17 | 12+ | 16+ |
| **Western Europe** | - | 3 - 6 | 4 - 9 | 7 - 18 | 11 - 25 | 16+ |
| **Eastern Europe-Russia** | 2 - 5 | 3 - 6 | 4 - 9 | 7 - 14 | 11 - 18 | 15+ |
| **China** | < 5 | 3 - 8 | 6 - 11 | 9 - 14 | 12 - 17 | >15 |

The articles may be offered for sale to the user in a variety of packaging, ranging from an individually packaged product to a jumbo carton box comprising more than one hundred articles.

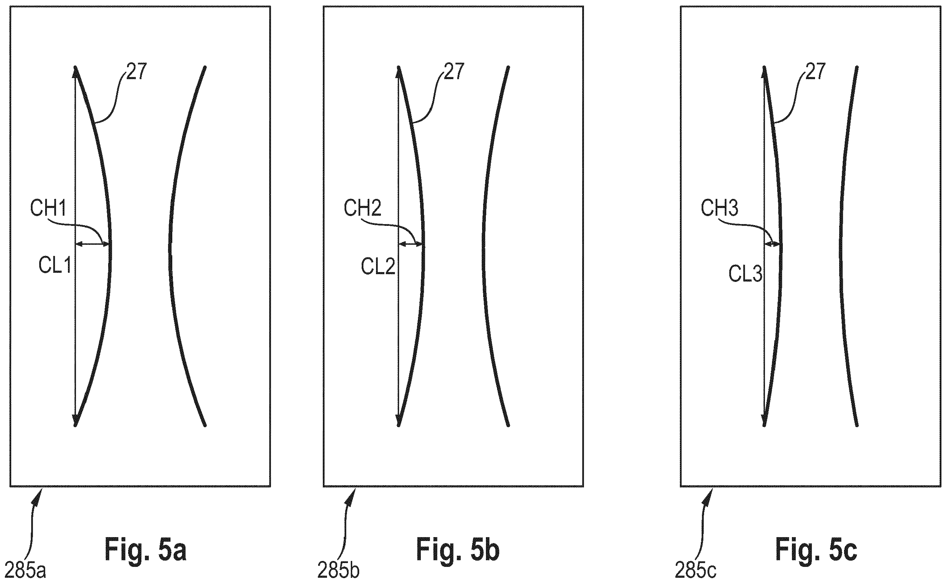

An outline of absorbent cores having channel-forming areas with different CL/CH ratios have been schematically represented in Fig. 5a,b,c and Fig. 6a,b,c. These cores may be for example used in articles having different sizes, to form an array of articles according to the invention, the absorbent article's size increasing from left to right. To more clearly illustrate the increasing ratio CL/CH from left to right, these cores have been represented with the same length and width, however it would be expected that the cores for the smaller size articles would be generally smaller in transversal and longitudinal dimensions. The ratio CL/CH for the core 285a shown on the left (CL1/CH1) is smaller than the ratio for the core 285b shown in the middle (CL2/CH2) which is itself smaller than the ratio for the core 285c disposed on the right hand side (CL3/CH3). In other words, everything being considered equal, the curves formed by the channel-forming areas become flatter as the size of the article increases.

Fig. 6a,b,c similarly illustrate a different type of curve for the channel-forming areas, wherein the core wrap bond of the core has a radius of curvature which is accentuated towards the back or front side edge of the core. This may be particularly useful to align or bring in proximity the point of the curve having the maximum channel height with the crotch point C' of the core. The general relation of the ratio CL/CH is conserved in this example (CL'1/CH'1 < CL'2/CH'2 < CL3'/CH3'), so that an array of product according to the invention may be provided by using these cores from left to right in absorbent products of increasing sizes. The same would of course apply for the core shown in Fig. 7 and Fig. 8.

Channel-forming areas provide bending lines that help improving fit and comfort of the article. Ideally, these bending lines should follow the natural curvature of the body, in particular at the areas joining the torso and the wearer thighs (so-called crotch line), which comprises high motion zone (towards the thighs) and low motion zone (towards the torso). This curvature changes with the age and weight of babies or body mass index for adults. By adjusting the length of the channel and its curvature ratio, it is possible to favor intentional folds in the absorbent core to contour of the baby legs around the crotch region of the wearer. While not wishing to be bound by theory, the inventors believe that the rate of baby growth (leg circumference) experience a larger morphological change for babies bellow 9 months than older babies, and estimated a ratio of growth versus diaper size in the order of 14 to 16% at early ages (up to 9 Kg babies) versus 6 to 10% for older babies.

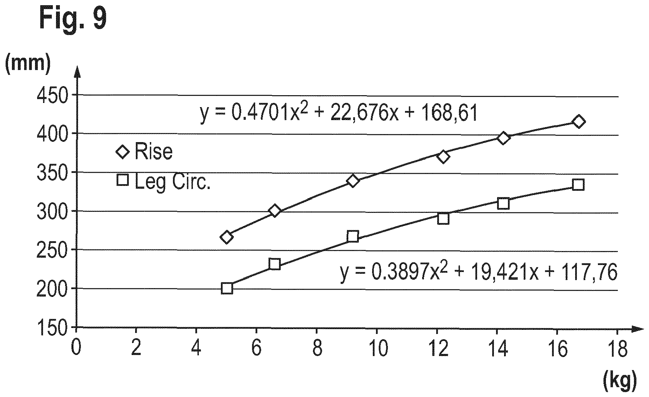

In addition, improved bending can be achieved when the channels extend from the crotch into the front and/or back region of the absorbent article, overreaching the transition region between the low and high motion area of the wearer's crotch line. It is believed that the Leg Hoop (defined by the oval circumference around the leg following crotch line) and Rise (defined as the distance from navel to equivalent high point at back of the baby) is also increasing in a higher rate for smaller babies than for larger babies. This relation is shown in Fig. 9 as a chart. It may also be advantageous that the channel's length increases similarly to the baby rise. In a first approximation, and while not wishing to be bound by these experimental figures, the following formula describing an advantageous channel length as a function of baby weight was found by the inventors: CL = -0.36M² + 17.5M + 62 (in mm), where M is the weight in kilo of the baby. The ratio CL/CH should also increase as a function of Leg Hoop and Rise, although it is more difficult to approximate accurately this increase as a function of weight. Also regional consumer habits and practices may affect the ideal perception of fit applications and in some instances.

### Example

The following table numerically illustrates an example of the dimensions (CL= channel length, CH = channel height) of channel-forming areas and how they can be varied among a line-up of absorbent articles of different sizes. The articles are baby diapers. The article's size / weight ranges relation is the one used at the time of filing for Pampers@ Bady-Dry® for size 2 to size 6, and Pampers@ New-Born for size 1 in WE.

| **Diaper's Size** | **1** | **2** | **3** | **4** | **5** | **6** |
|---|---|---|---|---|---|---|
| Weight range (kg) | 2-5 | 3-6 | 4-9 | 7-18 | 11-25 | >15 |
| CL (mm) | 138 | 159 | 197 | 227 | 227 | 254 |
| CH (mm) | 17 | 17 | 17 | 17 | 17 | 17 |
| Ratio CL/CH | 8.1 | 9.4 | 11.6 | 13.4 | 13.4 | 14.9 |
| % increase of the CL/CH ratio | - | 15 | 24 | 15 | 0 | 11 |

The % increase value shows the increase of the CL/CH ratio from one size to the next size. In this example, an array of article according to the invention is formed by the size 1, 2, 3 (or 4), 5 and 6. This example also illustrates that in some case the same channel design across more than one article's size offered (Size 4 and Size 5) may be acceptable, especially when the weight range of baby on those article sizes have a significant overlap or when the differences with the ideal channel dimensions are not justifying the cost and complexity associated with having multiple designs. Additional product sizes may be present in the line-up, in particular intermediate size such as Size 4+ or Size 5+ or for newborn (size 0 or "N"), as is known in the art. The line-up of articles may be for example sold under a given primary designation, which may typically be a tradename, for example Pampers@, and one or more secondary designation e.g. Baby-Dry®, New-Born, Active-Fit® or Swaddlers®. The weight ranges - article size relation indicated in the table above may be varied by the manufacturer, in particular for other product line-ups or geographies or times.

The amount of absorbent material, in particular superabsorbent polymer particles, present in the article may typically increases with the article's size. The amount of AGM will typically be varied by the manufacturer depending on the changing habits in the geographies considered, the absorbency and costs of the SAP used. As a purely indicative example, the following amount of commercially available SAP may be used in the cores described above.

| **Diaper's Size** | **1** | **2** | **3** | **4** | **5** | **S6** |
|---|---|---|---|---|---|---|
| **Amount of SAP (g) per core** | 6 | 6,5 | 9 | 12,5 | 13,5 | 14,8 |

### Test procedures

The values indicated herein are measured according to the methods indicated herein below, unless specified otherwise. All measurements are performed at 21°C ± 2°C and 50% ± 20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### Centrifuge Retention Capacity (CRC)

The CRC measures the liquid absorbed by the superabsorbent polymer particles for free swelling in excess liquid. The CRC is measured according to EDANA method WSP 241.2-05.

### Dry Absorbent Core Caliper Test

This test may be used to measure the caliper of the absorbent core (before use i.e. without fluid loading) in a standardized manner. This test is described in details in WO2014/093311, incorporated herein by reference.

### Absorbent Article Caliper Test

The Absorbent Article Caliper Test can be performed as for the Dry Absorbent Core Caliper Test with the difference that the caliper of the finished absorbent article is measured instead of the caliper of the core. More details for this test are also found in WO2014/093311.

### General

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so. Any feature or component described herein in relation with one embodiment may be combined with another feature or component of another embodiment unless indicated otherwise.

Unless indicated otherwise, the description and claims refer to the absorbent article, absorbent core or component thereof before use (i.e. dry, and not loaded with a fluid) and conditioned at least 24 hours at 21 °C +/- 2 °C and 50 +/- 20% Relative Humidity (RH).

Dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An array of at least two, preferably at least three, absorbent articles of different sizes, the articles having a wearer-facing side, a garment-facing side and an absorbent core (28) comprising an absorbent material (60) between the wearer-facing side and the garment-facing side,
each absorbent article notionally having a longitudinal axis (80), a front region (36), a back region (38) and an intermediate crotch region (37), each region having a third of the length of the article as measured along the longitudinal axis, wherein the absorbent core comprises a pair of channel-forming areas (26) at least partially present in the crotch region of the article, wherein these channel-forming areas are symmetrically disposed on opposite side of the longitudinal axis and are concave towards the longitudinal axis, wherein each channel-forming area has a length (CL) and a height (CH),
**characterized in that** the length to height ratio (CL/CH) of the channel-forming areas increases with the size of the articles,
**characterized in that** the absorbent material (60) of each article is enclosed by a core wrap (16, 16') and the channel-forming areas are formed by attaching the top side (288) of the core wrap to the bottom side of the core wrap (290) along a core wrap bond (27), and wherein the channel-forming areas comprise an area substantially free of absorbent material which is surrounded by absorbent material and through which the core wrap bond (27) is formed.

2. An array of absorbent articles according to the preceding claim wherein the length of the channel-forming areas increase with the article's size.

3. An array of absorbent articles according to any of the preceding claims wherein the length to height ratio of the articles of the array ranges from 5 to 40.

4. An array of absorbent articles according to any of the preceding claims wherein the length to height ratio increases at least by 10% between two following sizes of the array.

5. An array of absorbent articles according to any of the preceding claims wherein, for each article in the array, the channel-forming areas have a length (CL) which is between 25% and 90% of the length (L) of the article.

6. An array of absorbent articles according to any of the preceding claims, wherein the amount of absorbent material, in particular the amount of superabsorbent polymer, in each absorbent core increases with the size of the absorbent articles of the array.

7. An array of absorbent articles according to any of the preceding claims, wherein the absorbent core of each article of the array is substantially free of cellulose fibers.

8. An array of absorbent articles according to any of the preceding claims, wherein the absorbent material (60) of each absorbent core of the array comprises at least 90% by weight of superabsorbent polymer particles, in particular wherein all the absorbent material consists of superabsorbent polymer particles.

9. An array of absorbent articles according to claim 1, wherein the core wrap bond is formed by at least one of gluing, pressure bonding and/or ultrasonic bonding.

10. An array of absorbent articles according to any of the preceding claims, wherein the absorbent material is enclosed in a core wrap and further comprising a thermoplastic adhesive material that immobilizes at least some of the absorbent material within the core wrap, in particular an auxiliary glue (71, 72) on the inner side of the top side and/or bottom side of the core wrap, and/or a net of microfibrous glue applied on the absorbent material.

11. A line-up of articles comprising an array of absorbent articles according to any of the preceding claims.

12. A line-up of baby care diapers according to claim 11, wherein the line-up comprises at least five different articles having sizes segmented according to the weight of the baby.

13. A line-up according to the preceding claim, wherein the sizes of the articles of the line-up are selected from the groups consisting of:
- a first size, corresponding to a baby weight range whose boundaries encompass 4 kg;
- a second size, corresponding to a baby weight range whose boundaries encompass 5 kg;
- a third size, corresponding to a baby weight range whose boundaries encompass 8 kg;
- a fourth size, corresponding to a baby weight range whose boundaries encompass 12 kg;
- a fifth size, corresponding to a baby weight range whose boundaries encompass 14 kg;
- a sixth size, corresponding to a baby weight range whose boundaries encompass 17 kg.

14. A method of making an array of absorbent articles according to any of the preceding claims, comprising the steps:
- making at least two different absorbent cores, preferably at least three different absorbent cores, each having channel-forming areas as indicated in claim 1 and having at least two different length to height ratios;
- assembling the absorbent cores into absorbent articles of different sizes with the length to height ratios increasing with the size of the articles.

## Patentansprüche

1. Anordnung von mindestens zwei, vorzugsweise mindestens drei Absorptionsartikeln unterschiedlicher Größen, wobei die Artikel eine trägerseitige Seite, eine zum Kleidungsstück zeigende Seite und zwischen der trägerseitigen Seite und der zum Kleidungsstück zeigenden Seite einen ein Absorptionsmaterial (60) umfassenden Absorptionskern (28) aufweisen,
wobei jeder Absorptionsartikel gedacht eine Längsachse (80), eine vordere Region (36), eine hintere Region (38) und eine mittlere Schrittregion (37) aufweist, wobei jede Region, gemessen entlang der Längsachse, ein Drittel der Länge des Artikels ausmacht, wobei der Absorptionskem ein Paar kanalbildender Bereiche (26) umfasst, die zumindest teilweise in der Schrittregion des Artikels liegen, wobei diese kanalbildenden Bereiche symmetrisch auf einander entgegengesetzten Seiten der Längsachse angeordnet sind und zur Längsachse hin konkav sind, wobei jeder kanalbildende Bereich eine Länge (CL) und einer Höhe (CH) aufweist,
**dadurch gekennzeichnet, dass** das Länge-zu-Höhe-Verhältnis (CL/CH) der kanalbildenden Bereiche mit der Größe der Artikel zunimmt,
**dadurch gekennzeichnet, dass** das Absorptionsmaterial (60) jedes Artikels von einer Kernumwicklung (16, 16') umhüllt ist und die kanalbildenden Bereiche durch Befestigen der oberen Seite (288) der Kemumwicklung an der unteren Seite der Kernumwicklung (290) entlang einer Kernumwicklungsbindung (27) ausgebildet werden, und wobei die kanalbildenden Bereiche einen Bereich umfassen, der im Wesentlichen frei ist von dem Absorptionsmaterial, der umgeben ist von Absorptionsmaterial und durch den die Kernumwicklungsbindung (27) ausgebildet ist.

2. Anordnung von Absorptionsartikeln nach dem vorstehenden Anspruch, wobei die Länge der kanalbildenden Bereiche mit der Größe des Artikels zunimmt.

3. Anordnung von Absorptionsartikeln nach einem der vorstehenden Ansprüche, wobei das Länge-zu-Höhe-Verhältnis der Artikel der Anordnung im Bereich von 5 bis 40 liegt.

4. Anordnung von Absorptionsartikeln nach einem der vorstehenden Ansprüche, wobei das Länge-zu-Höhe-Verhältnis zwischen zwei aufeinander folgenden Größen der Anordnung um mindestens 10 % zunimmt.

5. Anordnung von Absorptionsartikeln nach einem der vorstehenden Ansprüche, wobei für jeden Artikel in der Anordnung die kanalbildenden Bereiche eine Länge (CL) aufweisen, die zwischen 25 % und 90 % der Länge (L) des Artikels beträgt.

6. Anordnung von Absorptionsartikeln nach einem der vorstehenden Ansprüche, wobei die Menge an Absorptionsmaterial, insbesondere die Menge an Superabsorberpolymer, in jedem Absorptionskem mit der Größe der Absorptionsartikel der Anordnung zunimmt.

7. Anordnung von Absorptionsartikeln nach einem der vorstehenden Ansprüche, wobei der Absorbtionskern jedes Artikels der Anordnung im Wesentlichen frei von Cellulosefasern ist.

8. Anordnung von Absorptionsartikeln nach einem der vorstehenden Ansprüche, wobei das Absorptionsmaterial (60) jedes Absorptionskerns der Anordnung zu mindestens 90 Gew.-% Superabsorberpolymerteilchen umfasst, wobei insbesondere das gesamte Absorptionsmaterial aus Superabsorberpolymerteilchen besteht.

9. Anordnung von Absorptionsartikeln nach Anspruch 1, wobei die Kernumwicklungsbindung durch mindestens eines von Kleben, Druckverbindung und/oder Ultraschallverbindung ausgebildet wird.

10. Anordnung von Absorptionsartikeln nach einem der vorstehenden Ansprüche, wobei das Absorptionsmaterial in einer Kernumwicklung umschlossen ist und ferner ein Thermoplast-Klebematerial umfasst, das mindestens einen Teil des Absorptionsmaterials innerhalb der Kernumwicklung immobilisiert, insbesondere einen Hilfsklebstoff (71, 72) auf der Innenseite der oberen Seite und/oder der unteren Seite der Kernumwicklung und/oder ein Gitter aus mikrofaserhaltigem Klebstoff, der auf das Absorptionsmaterial aufgebracht ist.

11. Aufreihung von Artikeln, umfassend eine Anordnung von Absorptionsartikeln nach einem der vorstehenden Ansprüche.

12. Aufreihung von Babywindeln nach Anspruch 11, wobei die Aufreihung mindestens fünf verschiedene Artikel mit Größen umfasst, die nach dem Gewicht des Babys gegliedert sind.

13. Aufreihung nach dem vorstehenden Anspruch, wobei die Größen der Artikel der Aufreihung ausgewählt sind aus den Gruppen bestehend aus:
- einer ersten Größe, die einem Babygewichtsbereich bis einschließlich 4 kg entspricht;
- einer zweiten Größe, die einem Babygewichtsbereich bis einschließlich 5 kg entspricht;
- einer dritten Größe, die einem Babygewichtsbereich bis einschließlich 8 kg entspricht;
- einer vierten Größe, die einem Babygewichtsbereich bis einschließlich 12 kg entspricht;
- einer fünften Größe, die einem Babygewichtsbereich bis einschließlich 14 kg entspricht;
- einer sechsten Größe, die einem Babygewichtsbereich bis einschließlich 17 kg entspricht.

14. Verfahren zum Herstellen einer Anordnung von Absorptionsartikeln nach einem der vorstehenden Ansprüche, umfassend die Schritte:
- Herstellen von mindestens zwei unterschiedlichen Absorptionskernen, vorzugsweise mindestens drei unterschiedlichen Absorptionskernen, die jeweils kanalbildende Bereiche aufweisen, wie in Anspruch 1 angegeben, und die mindestens zwei unterschiedliche Länge-zu-Höhe-Verhältnisse aufweisen;
- Zusammensetzen der Absorptionskerne zu Absorptionsartikeln unterschiedlicher Größen mit Länge-zu-Höhe-Verhältnissen, die mit der Größe der Artikel zunehmen.

## Revendications

1. Ensemble d'au moins deux, de préférence au moins trois articles absorbants de tailles différentes, les articles ayant un côté faisant face au porteur, un côté faisant face au vêtement et un noyau absorbant (28) comprenant un matériau absorbant (60) entre le côté faisant face au porteur et le côté faisant face au vêtement,
chaque article absorbant ayant théoriquement un axe longitudinal (80), une région antérieure (36), une région postérieure (38) et une région d'entrejambe (37) intermédiaire, chaque région composant un tiers de la longueur de l'article telle que mesurée le long de l'axe longitudinal, dans lequel le noyau absorbant comprend une paire de zones formant canal (26) au moins partiellement présentes dans la région d'entrejambe de l'article, dans lequel ces zones formant canal sont disposées symétriquement sur les côtés opposés de l'axe longitudinal et sont concaves en direction de l'axe longitudinal, dans lequel chaque zone formant canal a une longueur (CL) et une hauteur (CH),
**caractérisé en ce que** le rapport longueur sur hauteur (CL/CH) des zones formant canal augmente avec la taille des articles,
**caractérisé en ce que** le matériau absorbant (60) de chaque article est inclus dans une enveloppe de noyau (16, 16') et les zones formant canal sont formées en fixant le côté supérieur (288) de l'enveloppe de noyau sur le côté inférieur de l'enveloppe de noyau (290) le long d'une liaison d'enveloppe de noyau (27) et dans lequel les zones formant canal comprennent une zone essentiellement dépourvue de matériau absorbant, qui est entourée d'un matériau absorbant et à travers laquelle la liaison d'enveloppe de noyau (27) est formée.

2. Ensemble d'articles absorbants selon la revendication précédente, dans lequel la longueur des zones formant canal augmente avec la taille de l'article.

3. Ensemble d'articles absorbants selon l'une quelconque des revendications précédentes, dans lequel le rapport longueur sur hauteur des articles de l'ensemble va de 5 à 40.

4. Ensemble d'articles absorbants selon l'une quelconque des revendications précédentes, dans lequel le rapport longueur sur hauteur augmente au moins de 10 % entre deux tailles suivantes de l'ensemble.

5. Ensemble d'articles absorbants selon l'une quelconque des revendications précédentes, dans lequel, pour chaque article de l'ensemble, les zones formant canal ont une longueur (CL) qui est comprise entre 25 % et 90 % de la longueur (L) de l'article.

6. Ensemble d'articles absorbants selon l'une quelconque des revendications précédentes, dans lequel la quantité de matériau absorbant, en particulier la quantité de polymère superabsorbant, dans chaque noyau absorbant augmente avec la taille des articles absorbants de l'ensemble.

7. Ensemble d'articles absorbants selon l'une quelconque des revendications précédentes, dans lequel le noyau absorbant de chaque article de l'ensemble est sensiblement exempt de fibres de cellulose.

8. Ensemble d'articles absorbants selon l'une quelconque des revendications précédentes, dans lequel le matériau absorbant (60) de chaque noyau absorbant de l'ensemble comprend au moins 90 % en poids de particules de polymère superabsorbant, en particulier dans lequel tous les matériaux absorbants sont constitués de particules de polymère superabsorbant.

9. Ensemble d'articles absorbants selon la revendication 1, dans lequel la liaison d'enveloppe de noyau est formée par au moins l'un d'un collage, d'une liaison par pression et/ou d'une liaison par ultrasons.

10. Ensemble d'articles absorbants selon l'une quelconque des revendications précédentes, dans lequel le matériau absorbant est inclus dans une enveloppe de noyau et comprenant en outre un matériau adhésif thermoplastique qui immobilise au moins une partie du matériau absorbant au sein de l'enveloppe de noyau, en particulier une colle auxiliaire (71, 72) sur le côté interne du côté supérieur et/ou du côté inférieur de l'enveloppe de noyau et/ou un réseau de colle microfibreuse appliqué sur le matériau absorbant.

11. Gamme d'articles comprenant un ensemble d'articles absorbants selon l'une quelconque des revendications précédentes.

12. Gamme de couches de soins pour bébé selon la revendication 11, dans laquelle la gamme comprend au moins cinq articles différents ayant des tailles segmentées en fonction du poids du bébé.

13. Gamme selon la revendication précédente, dans laquelle les tailles des articles de la gamme sont choisies dans les groupes constitués par :
- une première taille, correspondant à une plage de poids du bébé, dont les limites englobent 4 kg ;
- une deuxième taille, correspondant à une plage de poids du bébé, dont les limites englobent 5 kg ;
- une troisième taille, correspondant à une plage de poids du bébé, dont les limites englobent 8 kg ;
- une quatrième taille, correspondant à une plage de poids du bébé, dont les limites englobent 12 kg ;
- une cinquième taille, correspondant à une plage de poids du bébé, dont les limites englobent 14 kg ;
- une sixième taille, correspondant à une plage de poids du bébé, dont les limites englobent 17 kg.

14. Procédé de fabrication d'un ensemble d'articles absorbants selon l'une quelconque des revendications précédentes, comprenant les étapes consistant à :
- réaliser au moins deux noyaux absorbants différents, de préférence au moins trois noyaux absorbants différents, chacun ayant des zones formant canal comme indiqué dans la revendication 1 et ayant au moins deux rapports longueur sur hauteur différents ;
- assembler les noyaux absorbants en articles absorbants de tailles différentes avec les rapports longueur sur hauteur augmentant avec la taille des articles.
